# EUROPEAN PATENT APPLICATION

(11) **EP 1 870 125 A1**
(43) Date of publication of application: **26.12.2007**
(21) Application number: 07000944.4
(22) Date of filing: 17.01.2007
(51) Int. Cl.: A61M 25/06

(54) **A separable medical device**

(30) Priority: 22.06.2006 US 473956
(71) Applicant: Kurth, Paul, Santa Barbara, CA 93108 (US); Mackley, Malcom R., 32 Chesterton Hall Crescent Cambridge CB4 1AP (GB); Hallmark, Bart, 28 Post Mill Gardens Grundisburgh IP13 6UP (GB)
(72) Inventor: Kurth, Paul, Santa Barbara, CA 93108 (US); Mackley, Malcom R., 32 Chesterton Hall Crescent Cambridge CB4 1AP (GB); Hallmark, Bart, 28 Post Mill Gardens Grundisburgh IP13 6UP (GB)
(74) Representative: Duxbury, Stephen

(57) **Abstract**

A medical apparatus comprises a separable introducer, sheath, catheter, cannula, or needle, each having a wall and a length; and at least one capillary defined within the wall of a hub or along the length of the introducer, sheath, catheter, cannula, or needle to provide a corresponding zone of separation therein.

## Description

### Field of the Invention

The invention relates to the field of separable medical devices.

### Description of the Prior Art

Separable medical devices are well known and typically include inter alia cardiac hemostatic valves and introducers, alone or in combination with each other. The reasons for desiring the separation of an introducer or medical device can be varied, but the most common usage is in connection with the removal of a first elongate instrument, such as a catheter or introducer, from a second elongate instrument, such as a guidewire, dilator, needle, catheter, pacemaker lead, another introducer or other medical device, when telescopic removal of the first instrument over the proximal end of the second instrument is not possible or convenient, the distal end of both the first and second instruments typically being at some point in time inserted within the body during a medical procedure. Endovascular procedures are the most common context of such usage, but the context includes endoscopic and other low invasive or noninvasive procedures as well.

Examples of such prior art separable devices are described in Lee U.S. Patents 5,125,904 and 5,312,355 in the case of combined hemostatic valves and introducers. Additional prior art examples of separable valves, introducers or devices are disclosed in Philip O. Littleford, et al, "The American Journal of Cardiology," Vol. 43, pp. 980-982 (May 1979); Littleford, U.S. Patent 4,166,469; 4,243,050 and 4,345,606; Osborne, Re. 31,855, a reissue of U.S. Patent 4,306,562; Boarini et al., U.S. Patent 4,411,654; Moorehead, U.S. Patent 4,983,168; Kousai et al., U.S. Patent 4,883,468; Haindl, German Patent 3140915; Heck, U.S. Patent 6,083,207; Lang, U.S. Patent 6,712,789, Pohndorf U.S. Patent 5,441,504 and many others, all of which are incorporated herein by reference. This listing of prior art separable devices is by no means comprehensive, but is illustrative of various endovascular applications using separable valves and introducers.

The various prior art devices allow for separation of an introducer or device by a plurality of different means, such as a preferred molecular orientation in the material being torn, a coupling or joint, a score line, a notch, a partial cut, a line of resiliently biased or compressed mechanical opening and sealing, or a molded line of relative weakness in the introducer or a wall of the device. All of these various mechanisms for allowing separation are referenced for the purposes of this specification as a "line of weakness". The introducer or device is then separated on the line of weakness by ripping, failing, tearing, splitting, opening, cracking, fracturing or some other action of material separation.

However, the choice of separation mechanism in the line of weakness in a separable medical device will be dictated by many factors, including suitability of the material to use of the separation mechanism and cost of manufacturing the medical device with the chosen separation mechanism in it. What is needed is a separation mechanism that can be cost effectively used in molded medical devices and in particular in extruded tubes.

### Brief Summary of the Invention

The illustrated embodiment is a separable medical apparatus comprising a wall and at least one capillary defined and enclosed within the wall to provide a zone of separation. The zone of separation comprises a thinning of the wall by presence of the capillary as compared to elsewhere in the wall where the capillary is not present.

In one embodiment the wall comprises a housing of a hemostatic valve, or housing of an introducer, sheath, catheter, cannula, or needle.

Another preferred embodiment is a film with an arcuate shaped cross-section having a capillary disposed therein. The distance between the film inner convex surface and the film outer convex surface defining the film wall thickness. The capillary is fully contained within the film wall thickness. The distance between the film inner convex surface and the capillary inner concave surface defining the film inner wall thickness. The distance between the capillary outer convex surface and the film outer convex surface defining the film outer wall thickness. Whereas the sum of the film inner wall thickness and the film outer wall thickness is less than the film wall thickness. Preferably, the sum of the film inner wall thickness and the film outer wall thickness is less than 75% of the film wall thickness. Most preferably, the sum of the film inner wall thickness and the film outer wall thickness is between 25% and 60% of the film wall thickness. The Film material may be a polymer, elastomer, or rubber. Preferably the film material is a thermoplastic material. Most preferably, the film material is from the group of polyolefins, polyamides or polyetherblockamides.

While the apparatus and method has or will be described for the sake of grammatical fluidity with functional explanations, it is to be expressly understood that the claims are not to be construed as necessarily limited in any way by the construction of "means" or "steps" limitations, but are to be accorded the full scope of the meaning and equivalents of the definition provided by the claims under the judicial doctrine of equivalents. The invention can be better visualized by turning now to the following drawings wherein like elements are referenced by like numerals.

### Brief Description of the Drawings

Fig. 1 is a perspective view of a medical device in which a pair of capillaries have been defined to form a zone of separation according to the invention.

Fig. 2 is a perpendicular cross sectional view through a tubular body, such as an introducer or catheter, devised according to another embodiment of the invention in which three capillaries are defined.

Fig. 3 is a perpendicular cross sectional view through a tubular body, such as an introducer or catheter, devised according to still another embodiment of the invention in which two capillaries define an angular segment which is peeled out of the tubular body.

Figs. 4a and 4b are partial cut away perspective views of a wall of uniform thickness of a medical device in which one or two capillaries are defined respectively. Fig. 4c is a partial cut away perspective view of a wall of nonuniform thickness of a medical device in which a capillary are defined.

Figs. 5a - 5e are partial cut away perspective views of a wall of a medical device which illustrate various different embodiments where the capillaries are defined in different patterns in the wall.

Fig. 6 is a diagram of a perspective view of a tubular body where two opposing capillaries are defined in the wall of the body in a double helix along the longitudinal length of the body.

Fig. 7 is a perpendicular cross sectional view through a tubular body showing a stiffener or wire disposed in one of the capillaries.

Fig. 8 is a diagram of an extrusion apparatus for manufacturing the body illustrated in Figs. 1-7.

Fig. 9 is a side cross sectional view of an extrusion die used in the apparatus of Fig. 8 to make the tubular body of Fig. 1.

Fig. 10 is a end plan view of the extrusion die of Fig. 9 as seen through section lines 10 -10 of Fig. 9.

The invention and its various embodiments can now be better understood by turning to the following detailed description of the preferred embodiments which are presented as illustrated examples of the invention defined in the claims. It is expressly understood that the invention as defined by the claims may be broader than the illustrated embodiments described below.

### Detailed Description of the Preferred Embodiments

The illustrated embodiment is illustrated in an extruded tube 10 used or usable in a medical apparatus such as a catheter or introducer. The invention may be employed in combination with any medical device now known or later devised in which the device or a component thereof is separable. It is to be expressly understood that the invention can be broadly applied in various types of medical devices 12 and need not be limited to molded devices nor to devices having a tubular component 10. Further, wherever reference is made to "separable", "separation", or "separate", it is to be understood for the purposes of this specification that any mechanism or process for dividing a body, whether it be opening the body on a single line or dividing a body on multiple lines, which mechanism or process is now known or later devised is included within the scope of meaning. For example, "separable", "separation", or "separate" is meant to include the concepts of peeling, shearing, splitting, cutting, ripping, tearing, fracturing, snapping, breaking, popping, exploding, failing, unzipping, unlatching, decoupling, coming apart, parting, opening, splaying, unfolding, uncurling, dividing, severing, sundering, detaching, disconnecting, unconnecting and all such similar concepts without limitation.

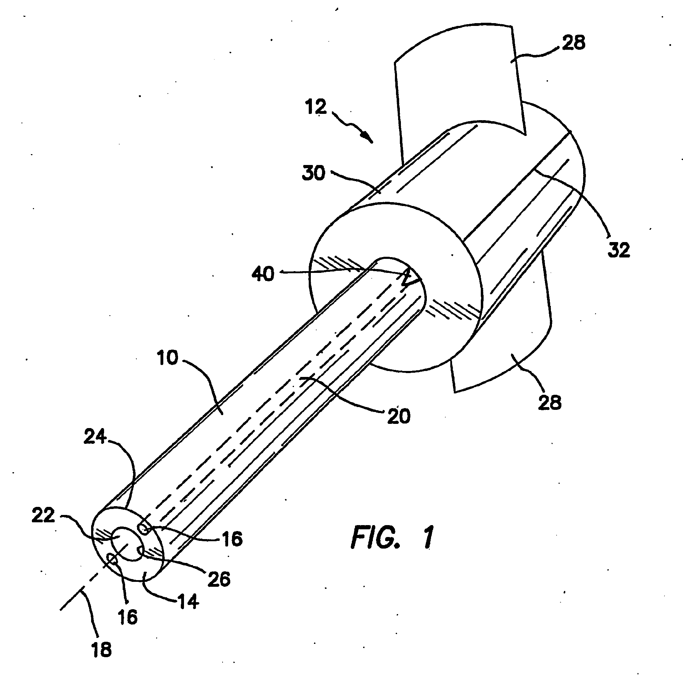

However, the invention is first illustrated as being embodied in a medical device 12 as diagrammatically shown in Fig. 1 comprising a tubular body 10 having a longitudinal axis and a wall 14 and at least one capillary 16 defined and enclosed within the wall 14 and extending along the length of the longitudinal axis 18 to provide a zone of separation 20 in body 10 indicated by imaginary dotted boundary lines to indicate only that the separation occurs somewhere in the zone between the dotted boundary lines.

In the embodiment of Fig. 1 two capillaries 16 are defined in wall 14 diametrically opposed to each other. In this embodiment there are two zones of separation 20 overlying and underlying each capillary 16 in the interstitial material of wall 14 between the inside surface 26 of a central lumen 22 defined along the longitudinal axis of body 10 and capillary 16 on one hand, and the also in the interstitial material of wall 14 between capillary 16 and the outside surface 24 of body 10 on the other hand.

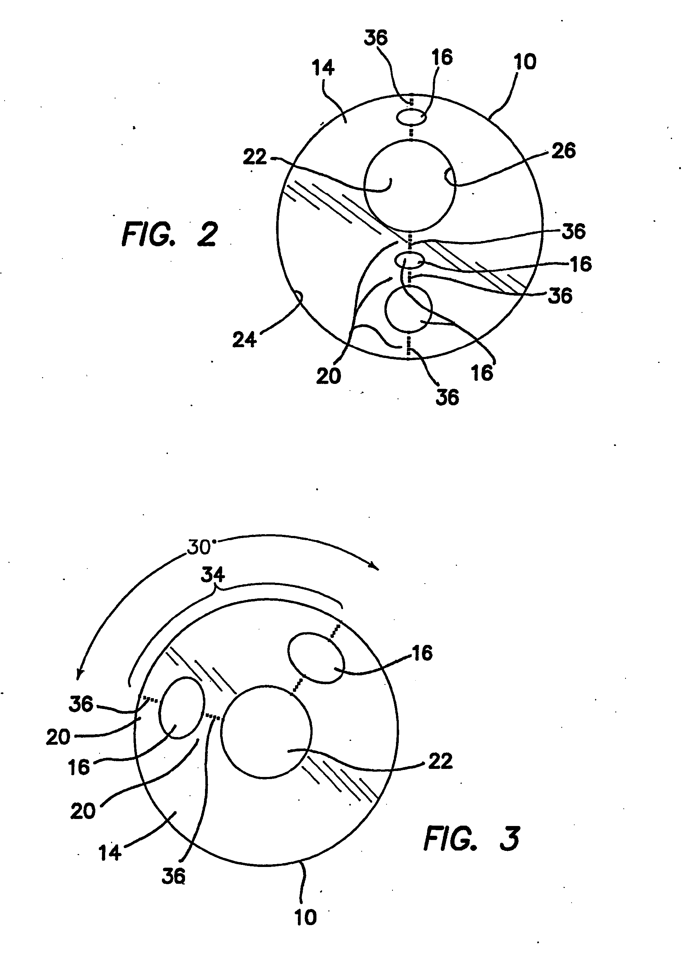

It must be understood that lumen 22 of body 10 need not be central, but could be defined off center or in any radial position within body 10. In this respect it is not to be assumed that where two or more capillaries 16 are defined in wall 14 that they are necessarily of equal radius nor circular in cross section. For example, as seen in the perpendicular cross sectional view of body 10 in Fig. 2, the invention includes embodiments where body 10 has an off center lumen 22 and multiple capillaries 16 of unequal size may be provided between surface 24 and 26 of body 10.

The illustrations of Figs. 1 and 2 have disproportionately enlarged the thickness of wall 14 in order to provide ease of visualization, but in the illustrated embodiments where the body 10 has a central lumen 22 wall 14 may have a wall thickness of approximately 100 µm to 2mm or more with a preferred wall thickness at 300µm for a tubular body and capillaries 16 with an average diameter of approximately 50 µm to 500 µm or more. Typically, capillaries 16 are not circular in cross section, but are generally ovulate or elliptical with a major axis diameter of the order of 50 - 600 µm, and preferably at 230 µm and a minor axis diameter of the order of 35 - 400 µm and preferably at 140µm in a 300µm thick tubular body. While capillaries 16 are often small enough to exhibit capillary behavior with water or other aqueous solutions, this is not a requirement of the invention and diameters large enough to only weakly show capillary action or not at all are contemplated as being within the scope of the invention.

In the preferred embodiment body 10 is fabricated as extruded tubing, but may also be fabricated as a continuous film with no edges in which film capillary 16 is defined. In one embodiment body 10 assumes the form of an extruded continuous film. Walls 14 of body 10 may be composed of polyethylene, polypropylene, fluorinated ethylene propylene (FEP), polyamide (PA), polyetherblockamide (PEBA), and in general any fluoropolymer, thermoplastic material, thermoplastic elastomer, thermoplastic vulcanate or thermoset material.

The illustrated embodiment of the invention may further comprise at least one handle 28 coupled to the tubular body 10 to facilitate separation of the tubular body 10 along the zone of separation 20 by manipulation of the handle 28. Fig. 1 shows an embodiment where a separable valve housing 30 is included as part of the overall medical apparatus with body 10 and a pair of handles 28 attached to the separable valve housing 30. The line of weakness 32 of housing 30 by whatever means devised is approximately aligned with the zone of separation 20 to allow the separation of valve housing 30 and body 10 in one operation by means of manipulation or pulling apart of handles 28. It is also contemplated that handles 28 may be directly coupled or attached to body 10 to facilitate separation of body 10 by pulling, particularly when body 10 is not combined with valve housing 30.

The medical device which is combined with the tubular body 10 is not limited to a valve housing 30 as described above, but is expressly meant to include a separable introducer, sheath, catheter, cannula, or needle or a hub for the same. In such embodiments at least one handle 28 may extend from the separable introducer, sheath, catheter, cannula or needle. The handle 28 may be singular and be itself separable in two sections or two or more handles 28 may be provided. The handle 28 is arranged and configured to separate the introducer, sheath, catheter, cannula or needle so that when the handle 28 is pulled apart in two sections, the handle 28 or each handle section is coupled to a different portion of the introducer, sheath, catheter, cannula or needle between the two zones of separation defined by the capillaries 16. The handle 28 is arranged and configured to separate the separable hemostatic valve housing 30 and the introducer, sheath, catheter, cannula or needle (body 10 in Fig. 1) in one operation by separating the introducer, sheath, catheter, cannula or needle along the zone of separation 20 when the handle 28 is pulled apart.

In such embodiments, the medical apparatus may further comprise a cut or notch 40 shown in Fig. 1 defined in a proximal end of the zone of separation 20 in the introducer, sheath, catheter, cannula or needle, represented diagrammatically by body 10 in Fig. 1, to facilitate starting of the separation of the introducer, sheath, catheter, cannula or needle along the zone of separation 20.

In the embodiment of Fig. 1 at least two capillaries 16 are defined in the wall 14 and extend along the length of the longitudinal axis 18, or more particularly exactly two capillaries 16 are defined in the wall 14 and preferably the two capillaries are diametrically opposed from each other. However, it is to be expressly understood that the two capillaries may be azimuthally offset by any angular measure, such as shown in the perpendicular cross sectional view of Fig. 3 where capillaries 16 are set approximately 30° apart from each other. In this case the angular segment 34 is effectively unzipped from body 10 by means of separation along the 30° offset zones of separation 20 corresponding to capillaries 16 and lumen 22 longitudinally splayed or opened along one side.

In any case, in the embodiments where a single capillary 16 is used to define a zone of separation 20, the zone of separation 20 is radially adjacent to the capillary 16 as indicated symbolically by the region in the locale of the dotted radial line segments 36 in Fig. 3. This region is defined as the capillary wall and the zone of separation is then defined in some path or paths through the thinnest shared portions of the walls 14 of the tubular body 10 and the capillary 16. In the embodiment of Fig. 2 the thinnest shared portions of the walls 14 of the tubular body 10 and the capillary 16 are defined in three separate regions along three radial line segments 36 along one side of wall 14 and are defined in two separate regions along two radial line segments 36 along the opposing side of wall 14.

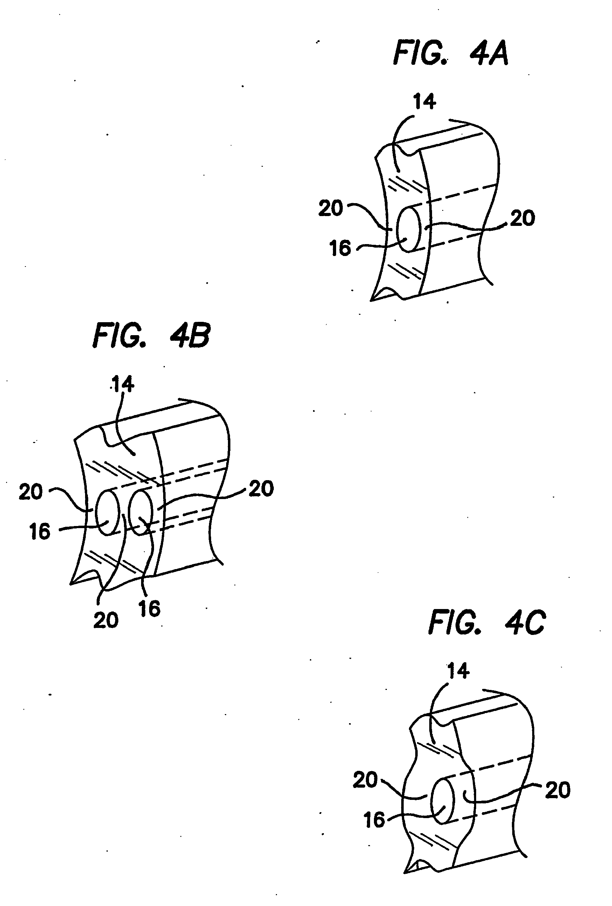

Fig. 4a illustrates an embodiment where wall 14, which need not be the wall of a tubular body, but a wall of any medical device without limitation, has an approximately uniform thickness in the neighborhood of capillary 16. Here the combined thickness of the two opposing zones of separation 20 is the difference between the uniform wall thickness and the diameter of capillary 16 in the direction perpendicular to the wall surface. The capillary wall thickness need not be half the difference, but may be asymmetrically divided.

Similarly, Fig. 4b illustrates the embodiment where wall 14 has an approximately uniform thickness in the neighborhood of two aligned capillaries 16. Here the combined thickness of the three aligned zones of separation 20 is the difference between the uniform wall thickness and the combined diameter of the two capillaries 16 in the direction perpendicular to the wall surface. Again the capillary wall thickness need not be a third of the difference, but may be asymmetrically divided.

Fig. 4c illustrates the embodiment where wall 14 has a nonuniform thickness in the neighborhood of a capillary 16. Wall 14 has been thickened in the embodiment of Fig. 4c above and below capillary 16. Here the combined thickness of the two aligned zones of separation 20 is the difference between the nonuniform wall thickness and the diameter of the capillary 16 in the direction perpendicular to the wall surface. The capillary wall thickness need not be half the difference, but may be asymmetrically divided. Fig. 4c shows a portion of the wall 14 between capillary 16 and the inner surface 26 of lumen 22 as an inner capillary wall 14a. The portion of the wall 14 between capillary 16 and the outer surface 24 of body 10 is termed the outer capillary wall 14b. It is intended that in the embodiment of Fig. 4c that the capillary wall thickness in each zone of separation 20 be less than the wall thickness of wall 14 of the body 10 adjacent to and laterally offset from capillary 16. In other words, while the combined thicknesses of capillary walls 14a and 14b may equal or exceed the thickness of wall 14 of tubular body 10, the thickness of the capillary walls 14a and 14b are each separately less than the thickness of wall 14 of tubular body 10. However, the preferred embodiment provides for a capillary wall thickness which is less than the adjacent wall thicknesses to capillary 16 to provide for one or more, and preferably two well defined and predictable zones of separation 20.

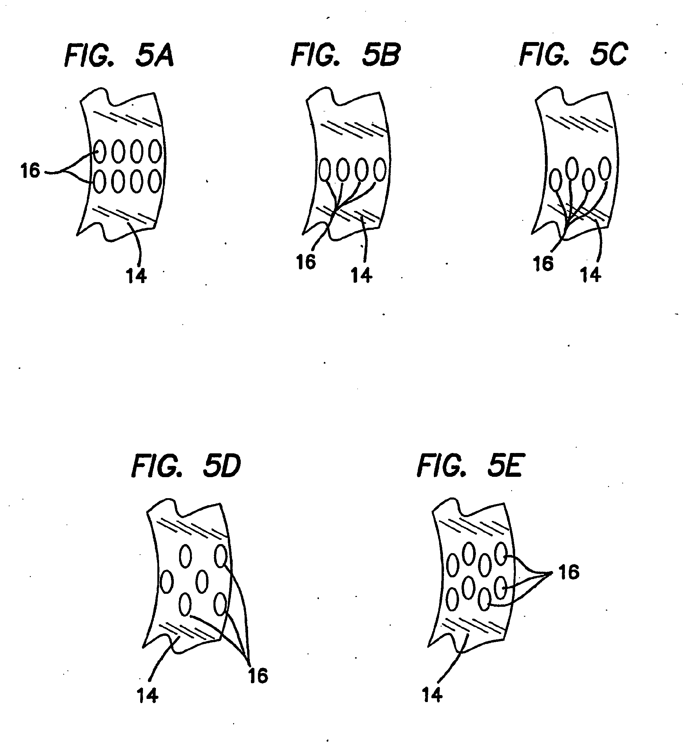

Thus, in general, it is to be expressly understood that a plurality of capillaries 16 may be provided in wall 14 and defined in a pattern in the zone or zones of separation 20. In the embodiment of Fig. 5a the pattern is a stack of capillaries 16 disposed across the thickness of the wall 14. In the embodiment of Fig. 5b the pattern is two or more aligned stacks of capillaries 16 disposed across the thickness of the wall 14. The embodiment of Fig. 5c is a pattern of staggered capillaries 16 or in particular two staggered lines of capillaries 16. The embodiment of Fig. 5d is a pattern of a plurality of staggered capillaries 16 or in particular three staggered lines of capillaries 16. The embodiment of Fig. 5e is a denser pattern of a plurality of staggered capillaries 16 than shown in Fig. 5d or in particular four staggered lines of capillaries 16.

In the preferred embodiment the material comprising body 10 is of such a nature that the zone of separation 20 comprises a line of tearing through the length of the capillary 16, preferably so that the capillary 16 is completely separated. By tearing it is meant that the zone of separation 20 begins to come apart at one location or end of the zone and then propagates continuously down the line of weakness until the entire capillary 16 is separated, much like unzipping a zipper according to the control of the manipulation of handles 28. However, it is entirely within the scope of the invention that the separation may be only partially propagated down the longitudinal axis 18 if desired or the separation may be more sudden, such as in a fracturing or snapping apart of the entire capillary 16.

Further, it is contemplated that a tool may also be employed to assist in the separation of capillary 16 by propagating the tearing or separation of the capillary. For example, an elongate tool can be disposed into the central lumen 22 or capillary 16, which tool is then used to expand lumen 22 or capillary 16 to stretch the capillary walls to tear or separate body 10.

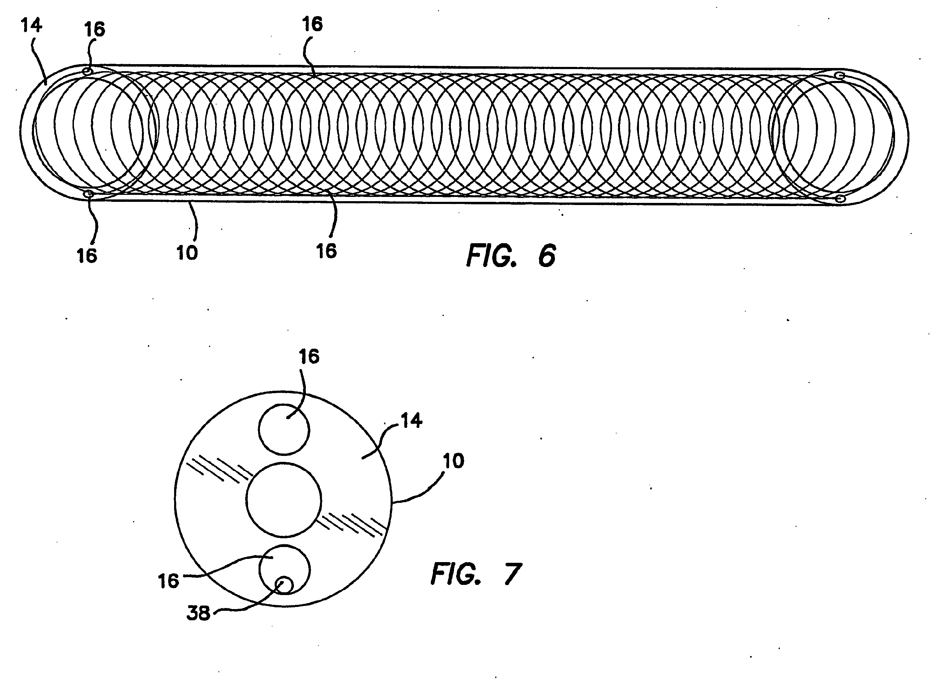

In any case, in the preferred embodiments there is a line of tearing along the length of the longitudinal axis which comprises two longitudinal, azimuthally aligned, linear or curvilinear lines of separation. In the embodiments of Figs. 1 and 2 the zone of separation 20 along the length of the longitudinal axis is defined at a single azimuthal position. However, the zone of separation need not be parallel to axis 18 but may assume any curvilinear shape according to the shape given to capillary 16. Fig. 6 illustrates the embodiment where a pair of diametrically opposed capillaries 16 are spiraled inside wall 14 of body 10 to form a double helix which defines the zones of separation 20.

It is to be further understood that a gas, liquid or solid may be employed to fill capillary 16 in whole or part. For example, a stiffener or wire 38 as shown in the side cross sectional view of Fig. 7 may be disposed in capillary 16 to provide a means for shaping body 10 or providing a predetermined degree of resiliency where the material of body 10 otherwise has none.

In addition to the various structural embodiments described above the illustrated embodiment of the invention also encompasses a method for separating a tubular body 10 having a longitudinal axis and a wall 14 comprising the steps providing at least one capillary 16 defined and enclosed within the wall 14 of the tubular body 10, wherein the capillary 16 extends along the length of the longitudinal axis 18 and defines a zone of separation 20. The method applies a stress across the zone of separation 20 to cause the tubular body 10 to separate along the zone of separation 20. The method further comprises the step of manipulating at least one handle 28 coupled to the tubular body 10 to facilitate separation of the tubular body 10 along the zone of separation 20.

In one embodiment the step of applying the stress across the zone of separation comprises separating the wall radially adjacent to the capillary. This usually means separating the capillary wall through the thinnest shared portions of the walls of the tubular body 10 and the capillary 16. Preferably, applying a stress across the zone of separation 20 causes tearing along a line through the length of the capillary 16, which completely separates the capillary 16 along a tear. The tearing comprises completely separating the capillary 16 along two longitudinal, azimuthally aligned, linear or curvilinear lines. Usually this means separating the capillary 16 along the length of the longitudinal axis 18 at a single azimuthal position.

In one embodiment the method comprises the steps of providing a separable introducer, sheath, catheter, cannula, needle, hub or hemostatic valve, all symbolically denoted as element 30 in Fig. 1, with at least one capillary 16 longitudinally defined therein to define a zone of separation 20. A stress is applied across the zone of separation 20 to cause the material of introducer, sheath, catheter, cannula, needle, hub or hemostatic valve in which the zone of separation 20 is defined to separate. Where two spaced-apart capillaries 16 are defined in the separable introducer, sheath, catheter, cannula, needle, hub or hemostatic valve, applying a stress across the zone or zones of separation 20 defined by each of the two capillaries 16 separates the introducer, sheath, catheter, cannula, needle, hub or hemostatic valve into two portions. This operation is facilitated by providing a pair of aligned handles or a separable handle 28 extending from the introducer, sheath, catheter, cannula, needle, hub or hemostatic valve. The handles or handle 28 is aligned with respect to the zone of separation 20 and manipulated to separate the introducer, sheath, catheter, cannula, needle, hub or hemostatic valve along the zones of separation 20 when the handles or sections of the handle 28 are pulled apart.

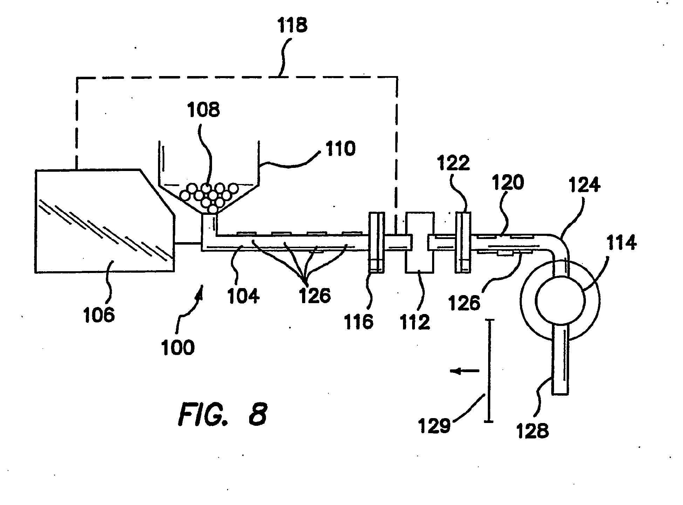

The structure of the illustrated embodiment and the methods by which the embodiments are separated having been described, the method of fabrication of the illustrated embodiments is disclosed generally in the incorporated applications PCT/GB2005/003084 published as W02006/016128 and PCT/GB2004/005196 published as W02005/056272. However, for the sake of clarity the preferred methods will be briefly summarized. Fig. 8 shows extrusion apparatus 100 for creating an extruded product or tube 10 having capillaries 16 defined in the walls 14 of tube 10. The apparatus 100 comprises screw extruder 104 driven by a motor 106. Extrudable material 108 is fed to the extruder screw 104 through a hopper 110. As the extrudable material 108 passes through the extruder screw 104 the material is melted to form a melt. The extruder screw 104 feeds the melt to a gear pump 112 which maintains a substantially constant flow of melt towards a die 114. The gear pump 112 is connected to the extruder screw 104 by a flange 116 which includes a screen filter to remove impurities from the melt flow. The motor 106 is controlled using a pressure feedback link 118 between the inlet of the gear pump 112 and the motor 106. The melt passes to the die 114 through an extruder barrel 120 which is connected to the gear pump 112 by a flange 122.

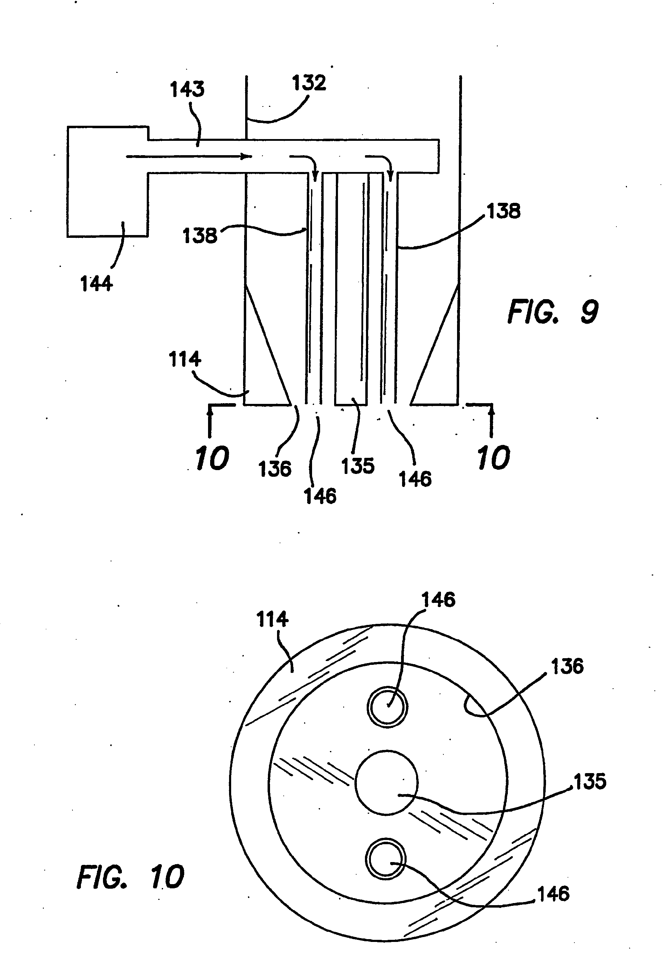

In this embodiment the extruder barrel 102 includes a 90° bend 124. Band heaters 126 are used to control the temperature at different stages in the extrusion apparatus 100. Band heaters 126 may be located within the extruder 100, on the flanges 116/122 on the gear pump 112, on the extruder barrel 120 and also on the die 114. The detail of the arrangement of the die 114 are shown in greater detail in Figs. 9 and 10. The melt passes through the die 114 and is formed into the desired shape and cross section. As the melt passes out of the die 114 it becomes an extruded body 10.

Fig. 9 shows a schematic side cross sectional view of die 114 of Fig. 8. The die 114 includes an entry portion 132, a convergent portion 134 and an orifice 136 which has a predetermined outer shape. The melt enters the entry portion 132 of the die 114, is gradually shaped by the convergent portion 134 until the melt exits the orifice 136. The die 114 further includes needles 138, only one of which is shown in Fig.9, positioned therein. The needle 138 includes a body portion 140 having a conduit 142 defined therein which is connected to a fluid source 144 by means of a second conduit 143 passing through a wall of the die 114 around which the melt must flow to pass to the orifice 136. The needle 138 further includes an outlet 146 at an end 148 of the needle 138. The needle 138 is arranged such that the outlet 146 is located within the orifice 136.

Fig. 10 is a schematic view of one embodiment of the die 114 from below for forming the body 10 of Fig. 1. Fig. 10 shows that the orifice 136 has a circular outer shape. The orifice 136 has a center post 135 to define lumen 22 and two needles 138 to define capillaries 16. In this example, the die includes two needles 138 with the outlets 146 distributed diametrically opposite from each other within the orifice 136. The pattern of needles 138 in orifice 136 may be modified to provide any pattern of capillaries now known or later devised including those shown in Figs. 4a- 4c, and 5a - 5e.

The process of fabrication of body 10 thus proceeds as follows. A polymer melt is produced in a screw extruder 104 and its resultant flow rate stabilized by means of a gear pump 112. This melt is then fed into a die 114 in the orifice of which is arranged a plurality of outlets 146 of needles 138 in a predetermined pattern. A conduit 142 through each needle 138 is fed from a horizontally orientated feed conduit 143, the entrance of which is open to atmosphere outside of the die 114 which is the fluid source 144.

The resulting extruded tube 10 is then passed over a series of rollers into a haul-off device (not shown) . The speed of the haul-off device can be altered so that tubes 10 with differing draw ratios can be produced. The die 114 is designed such that the incoming flow from the extruder 100, which is contained in a circular pipe of a different diameter than orifice 136, and which is altered such that it may pass through the orifice 136 of the die 114. The die 114 must effect this geometry change, and this is currently achieved by using a convergent die 114. The die 114 is also designed so that the flow over the pattern of needles 138 is substantially even. An even melt flow around the needles 138 facilitates creation of well formed body 10.

The process is operated at about 165°C using linear low density polyethylene (LLDPE). Other materials will require different temperatures according to conventional principles. The motor 106 is controlled using a pressure feedback loop that is set to 300PSI and this, in turn, causes a pressure of around a few bar in the die 114. Air is entrained as a result of the polymer flow over the pattern of needles 138 and the feed to this pattern is left open to the atmosphere.

The tear mechanism is disclosed generally in the incorporated application PCT/GB2005/003084 published as W02006/016128, which is now summarized for clarity. It has been noted that when body 10 is prepared with thin film walls 14, tearing body 10 by pulling apart the two sides by hand at the rate at which one normally tears a piece of paper, body 10 splits into two parts along the capillary 16 and the edges of the two parts are fairly straight. However, when the body 10 is pulled apart at rates of 10 mm/s or less, the edges of the two parts into which the film is split are curvy and have a wavy edge. It has been found that the force required to tear a thin walled body 10 is different depending on the mode of tearing.

A Texture Analyzer manufactured by Stable Micro Systems was used to measure the force required to tear thin planar walls 14. The force required to tear a thin wall 14 quickly was measured by clamping one end of the wall 14 to the Texture Analyzer and pulling the other end by hand. The force required to tear the wall 14 slowly was measured by clamping both ends to the Texture analyzer and pulling them apart at a rate of 10 mm/s. The force required to tear quickly a wall 14 having a single capillary 16 was fairly constant and close to 2 Newton. The force required to tear such a typical thin wall 14 slowly varied between about 1 and 9 Newton. When the wall 14 was torn slowly, it was observed that a web of stretched material formed in the region where the two sides of the wall 14 were being pulled apart. As the web grew the force required to tear the wall 14 increased. Eventually, the web would break and the force would drop abruptly to its low value from which it would again increase as a new web formed. In some cases, as the wall 14 was torn slowly, the tear propagated away from the zone of separation 20 and the wall 14 split to one side. To ensure that the tear propagates along the zone of separation 20 it is important that the reduction in the cross section in the zone of separation 20 is appropriate for the anticipated tear speed and force given the wall thickness and material properties from which it is made.

It is proposed that this difference in the tearing mechanisms occurs due to the changes in the stress/strain curves for materials dependant upon the speed of the application of the strain. It is also noted that, in some cases it is difficult to initiate the tearing by hand and some assistance of initiation was required. This usually involved forming a slit, notch or cut 40 along the zone of separation 20 from which to initiate the tear.

Ideally, in order to have good tearing characteristics, there should be a rapid transition between a thinned region of body 10 in the zone of separation 20 and a thicker region of body 10 away from capillary 16 so that the stress concentration causes the thinned region to reach the fracture point of the extruded material before the thicker region of the material begins to plastically deform. In the stress/strain curve for most materials there is a stress barrier that must be overcome before plastic deformation occurs. Ideally the shape of the zone of separation 20 is such that at the anticipated tear speed, the fracture point of the material is reached within the zone of separation 20 before the stress in an adjacent thicker region increases above the stress barrier for plastic deformation. The shape of the transition, the difference in cross sectional area of body 10 and the tear speed all have an effect on the mode of tearing.

Many alterations and modifications may be made by those having ordinary skill in the art without departing from the spirit and scope of the invention. Therefore, it must be understood that the illustrated embodiment has been set forth only for the purposes of example and that it should not be taken as limiting the invention as defined by the following invention and its various embodiments.

Therefore, it must be understood that the illustrated embodiment has been set forth only for the purposes of example and that it should not be taken as limiting the invention as defined by the following claims. For example, notwithstanding the fact that the elements of a claim are set forth below in a certain combination, it must be expressly understood that the invention includes other combinations of fewer, more or different elements, which are disclosed in above even when not initially claimed in such combinations. A teaching that two elements are combined in a claimed combination is further to be understood as also allowing for a claimed combination in which the two elements are not combined with each other, but may be used alone or combined in other combinations. The excision of any disclosed element of the invention is explicitly contemplated as within the scope of the invention.

The words used in this specification to describe the invention and its various embodiments are to be understood not only in the sense of their commonly defined meanings, but to include by special definition in this specification structure, material or acts beyond the scope of the commonly defined meanings. Thus if an element can be understood in the context of this specification as including more than one meaning, then its use in a claim must be understood as being generic to all possible meanings supported by the specification and by the word itself.

The definitions of the words or elements of the following claims are, therefore, defined in this specification to include not only the combination of elements which are literally set forth, but all equivalent structure, material or acts for performing substantially the same function in substantially the same way to obtain substantially the same result. In this sense it is therefore contemplated that an equivalent substitution of two or more elements may be made for any one of the elements in the claims below or that a single element may be substituted for two or more elements in a claim. Although elements may be described above as acting in certain combinations and even initially claimed as such, it is to be expressly understood that one or more elements from a claimed combination can in some cases be excised from the combination and that the claimed combination may be directed to a subcombination or variation of a subcombination.

Insubstantial changes from the claimed subject matter as viewed by a person with ordinary skill in the art, now known or later devised, are expressly contemplated as being equivalently within the scope of the claims. Therefore, obvious substitutions now or later known to one with ordinary skill in the art are defined to be within the scope of the defined elements.

The claims are thus to be understood to include what is specifically illustrated and described above, what is conceptionally equivalent, what can be obviously substituted and also what essentially incorporates the essential idea of the invention.

## Claims

1. A medical device comprising:
- a body having a longitudinal axis and a wall,
- a capillary extending through the body, and,
- a separation zone arranged between the wall of the body and the capillary.

2. The medical apparatus according to claim 1, wherein the separation zone comprises a thinning arranged between the body wall and the capillary.

3. The medical device according to any of the preceding claims, wherein the body takes the form of a film or of a tubular body, preferably comprising a continuous wall with no edges.

4. The medical device according to any of the preceding claims, comprising at least two capillaries.

5. The medical device according to claim 4, wherein the two capillaries are diametrically opposed from each other.

6. The medical device according to claims 4 or 5, wherein one or more of the separation zones are arranged radially adjacent to one or more of the capillaries.

7. The medical device according to any of the preceding claims, wherein the zone of separation forms a line of tearing through the length of the capillary.

8. The medical device of claim 8, wherein the capillary is separable along the line of tearing.

9. The medical device of claims 7 or 8, where the line of tearing along the length of the longitudinal axis comprises two longitudinal, azimuthally aligned, linear or curvilinear lines of separation.

10. The medical device according to any of the preceding claims 1-8, wherein the zone of separation along the length of the longitudinal axis is defined at a single azimuthal position.

11. The medical device according to any of the preceding claims, further comprising a gas, liquid or solid filling the capillary in whole or part.

12. The medical device according to claim 11 where the solid comprises a stiffener, which stiffener preferably comprises a wire.

13. The device according to any of the preceding claims, further comprising a lumen.

14. The medical device according to any of the preceding claims, further comprising at least one handle coupled to the body to facilitate separation of the body along the zone of separation by manipulation of the handle.

15. The medical apparatus according to claim 14, wherein the handle comprises two handgrips and whereby the handle is able to be pulled apart by pulling of the handgrips.

16. The medical apparatus according to claim 15, wherein each handgrip is coupled to a different portion of the body, which portions are arranged with different separation zones.

17. The medical device according to any of the preceding claims, wherein the medical device comprises one or more of the following: a separable introducer, a separable sheath, a separable catheter, a separable cannula, a separable needle, a separable hemostatic valve.

18. The medical device according to any of the preceding claims, being a medical introducer.

19. The medical device according to any of the preceding claims 1-17, being selected from one or more of the following: a medical introducer, a sheath, a catheter, a cannula, a needle, a hemostatic valve.

20. The medical device according to any of the preceding claims, wherein the body is formed by extrusion, and wherein the one or more capillaries and the one or more separation zones are also formed during extrusion of the body.

21. The medical device according to any of the preceding claims, wherein, when the extruded body is tubular, the tubular body is combined with the separable hemostatic valve as a separable introducer.

22. The medical device according to claim 21, wherein the handle is attached to the hemostatic valve.

23. The medical device according to any of the preceding claims, wherein the film has an arcuate shaped cross section, whereby the capillary is disposed therein.

24. The medical device according to claim 23, wherein a distance between an inner convex surface of the film and an outer convex surface of the film defines a film wall thickness.

25. The medical device according to claim 24, wherein the capillary is fully contained within the film wall thickness.

26. The medical device according to claims 24 or 25, wherein a distance between the film inner convex surface and a capillary inner concave surface defines an inner wall thickness of the film.

27. The medical device according to claims 24 or 25 or 26, wherein a distance between a capillary outer convex surface and the film outer convex surface defines an outer wall thickness of the film.

28. The medical device according to claim 27, wherein the sum of the film inner wall thickness and the film outer wall thickness is less than the film wall thickness.

29. The medical device according to claim 28, wherein the sum of the film inner wall thickness and the film outer wall thickness is less than 75 % of the film wall thickness, preferably wherein the sum of the film inner wall thickness and the film outer wall thickness is lies in the range of 25 %- 60 % of the film wall thickness.

30. The medical device according to any of the preceding claims, wherein the film material is a polymer, elastomer, or rubber, and is preferably a thermoplastic material, and is most preferably selected from the group of polyolefins, polyamides or polyetherblockamides.

31. The medical device according to any of the preceding claims, obtainable by providing an extrusion apparatus comprising an extruder having an inlet, a die including an orifice having a predetermined outer shape, a plurality of needles each having a body including an internal conduit for fluid flow, each needle further comprising an outlet from the internal conduit at an outlet end, the outlet end of each needle being arranged in a predetermined pattern substantially within the orifice of the die, the conduit of each needle being fluidly connected to a fluid source, feeding extrudable material into the extruder through the inlet, using the extruder to force the extrudable material towards the die and through the orifice in the die to produce an extrudate having a predetermined outer shape, using the needles to allow fluid to be drawn from the fluid source through the conduit to be entrained in the extrudate product to form the capillaries such that the extrudate includes capillaries therealong in the predetermined pattern.

32. The medical device according to any of the preceding claims, comprising a plurality of capillaries defined in a pattern in the zone of separation.

33. The medical device of claim 32, wherein the body wall has a thickness and wherein the pattern is a stack of capillaries disposed across the thickness of the wall.

34. The medical device according to claims 32 or 34, wherein the stack of capillaries comprises a plurality of linearly aligned capillaries.

35. The medical device according to any of the preceding claims, further comprising a cut or notch defined in a proximal end of the zone of separation in the body, to facilitate initiating the separation of the body along the zone of separation.

36. The medical device according to any of the preceding claims, wherein the body wall comprises one or more of the following: a housing of a hemostatic valve, a housing of an introducer, sheath, catheter, cannula, needle.

37. A medical assembly comprising: a medical device according to any of the preceding claims, the device being a medical introducer and a medical appliance introduceable by the medical introducer to a pre-determined medical utility site, at which site the medical introducer is separable, and removable from the medical appliance.

38. A medical assembly according to claim 37, wherein the medical appliance is selected from the group comprising: a sheath, a catheter, a cannula, a needle, a hemostatic valve.

39. A method for separating a medical device or assembly body as referred to in any of the preceding claims by applying a stress across the zone of separation to cause the body to separate along the zone of separation.

40. The method according to claim 39, further comprising manipulating at least one handle coupled to the body to facilitate separation of the tubular body along the zone of separation.

41. The method according to claim 40 where applying the stress across the zone of separation comprises separating the wall radially adjacent to the capillary.

42. The method according to claims 39 or 40, where applying the stress across the zone of separation comprises separating a capillary wall through the thinnest shared portions of the walls of the tubular body and the capillary.

43. The method according to any of the claims 39-42, where applying the stress across the zone of separation comprises tearing along a line through the length of the capillary.

44. The method according to any of the preceding claims 39-43, where tearing through the length of the capillary comprises completely separating the capillary along a tear.

45. The method according to any of the claims 39-44 where tearing through the length of the capillary comprises completely separating the capillary along two longitudinal, azimuthally aligned, linear or curvilinear lines.

46. The method according to any of the preceding claims 39-45, where applying the stress across the zone of separation comprises separating along the length of the longitudinal axis at a single azimuthal position.

47. Method of arranging a medical appliance, such as a sheath, a catheter, a cannula, a needle, a hemostatic valve, at a predetermined utility site comprising the step of utilizing a medical introducer according to claim 18 to arrange the medical appliance at the utility site.

48. Method according to claim 47, wherein the introducer is subsequently separated according to any of the claims 39-46.

49. Method according to claim 48, wherein the introducer is subsequently removed from the utility site.
